# EUROPEAN PATENT APPLICATION

(11) **EP 2 789 631 A1**
(43) Date of publication of application: **15.10.2014**
(21) Application number: 13305468.4
(22) Date of filing: 10.04.2013
(51) Int. Cl.: C07K 16/28, C07K 7/08, C07K 11/00, A61K 38/10

(54) **Compounds and methods for inhibition of binding of ICAM-4 to platelet integrin alphaIIbbeta3**

(71) Applicant: Synapse B.V., 6229 ER Maastricht (NL)
(72) Inventor: De Laat, Henricus Bastiaan, 6214 AE Maastricht (NL); Du, Xiaoyan, 3584 CX Utrecht (NL); Ruggeri, Zaverio M., La Jolla, CA 92037 (US)
(74) Representative: Desaix, Anne

(57) **Abstract**

The present invention relates to compounds and methods for inhibition of binding of ICAM-4 to platelet integrin α_{IIb}β₃.

The present invention also relates to a screening method and to a kit to detect or monitor *in vitro* the effect of a substance, drug or pharmaceutical agent on the ICAM-4/α_{IIb}β₃ interaction in a biological sample, while simulating blood flow conditions existing *in vivo.*

It concerns in particular an anti-ICAM-4 antibody or a fragment thereof capable of blocking ICAM-4 binding to platelet integrin α_{IIb}β₃, or an ICAM-4 mimetic peptide blocking ICAM-4 binding to platelet integrin α_{IIb}β₃, or an anti-CD61 antibody or a fragment thereof capable of blocking ICAM-4 binding to platelet integrin α_{IIb}β₃, for use in the treatment of a thrombotic disease, and for the preparation of pharmaceutical compositions for such treatment.

## Description

The present invention relates to compounds and methods for inhibition of binding of ICAM-4 to platelet integrin α_{IIb}β₃.

The present invention also relates to a screening method and to a kit to detect or monitor *in vitro* the effect of a substance, drug or pharmaceutical agent on the ICAM-4/α_{IIb}β₃ interaction in a biological sample, while simulating blood flow conditions existing *in vivo.* It concerns in particular an anti-ICAM-4 antibody or a fragment thereof capable of blocking ICAM-4 binding to platelet integrin α_{IIb}β₃, or an ICAM-4 mimetic peptide blocking ICAM-4 binding to platelet integrin α_{IIb}β₃, or an anti-CD61 antibody or a fragment thereof capable of blocking ICAM-4 binding to platelet integrin α_{IIb}β₃ for use in the treatment of a thrombotic disease, and for the preparation of pharmaceutical compositions for such treatment.

Haemostasis is a balance between prothrombotic and bleeding state. Components of haemostasis can be divided into a plasma part which is a solution derived from blood containing the coagulation proteins, and a cellular part of blood containing blood cells such as platelets, erythrocytes and leucocytes. *In vivo* interaction of the vessel with the haemostatic system prevents excessive blood loss when damage to a vessel occurs. One of the central coagulation proteins in the haemostatic system is thrombin. Thrombin is formed at the end of the coagulation cascade and causes fibrinogen to be converted into fibrin. The formed fibrin forms a tight mesh capturing platelets and erythrocytes thus giving rise to a plug that seals off the vessel. The measurement of thrombin generation is known to be indicative for either a prothrombotic or a bleeding phenotype and the formation of fibrin has been shown to be indicative of a bleeding phenotype related to point of care devices during surgery. Blood clot firmness is an important functional parameter for haemostasis *in vivo,* as a clot must resist blood pressure and shear stress at the site of the injury.

Thrombotic diseases, such as coronary infarction, stroke, pulmonary embolism, bleeding disorders as well as other disorders of the haemostatic system remain one of the main causes of mortality in Western society. Thus it remains important to keep understanding the mechanism involved in haemostasis and coagulation.

Erythrocytes are the major cellular components of blood. Their primary function is to transport oxygen to organs and carbon dioxide to the lungs. In the field of haemostasis which primarily involves endothelial cells, platelets and coagulation factors, erythrocytes are generally considered as passive participants. Erythrocytes have thus been shown to contribute to primary haemostasis, predominantly due to their rheological properties. It has accordingly been reported that prolonged bleeding can be successfully treated by elevated red blood cell count (Ho, CH. et al., Transfusion, 1996, 36:290*;* Ho, CH. et al., Blood, 1998, 91:1094*;* Livio M. et al., Lancet, 1982, 2:1013-1015). Abnormally high red blood cell count can predispose a patient to thrombotic disease (Boneu B. et al., Nouv. Rev. Fr. Hematol., 1994, 36:183-185*;* Rossi C. et al., J. Intern. Med., 1998, 244:49-53)*.* Erythrocytes have also been shown to be involved during secondary haemostasis by their passive entrapment inside the fibrin net, thereby stabilising the developing thrombus. Hematocrit together with hemoglobin and red blood cell count have been identified as risk factors for venous thrombosis in a general population (Braekkan S.K. et al., Haematologica, 2010, 95:270-275*).* Noteworthy, in the last decade, more evidences have surfaced to support an active role for erythrocytes in homeostasis. Erythrocytes were demonstrated to be able to interact with platelets (Santos M.T. et al., J. Clin. Invest., 1991, 87:571-580*;* Santos M. T. et al., Circulation, 1997, 95:63-68*;* Santos M.T. et al., J. Thromb. Haemost., 2008, 6:615-621*;* Valles J. et al., Blood, 1991, 78:154-162*;* Valles J. et al., Blood, 2002, 99:3978-3984)*.* In particular, it was shown that erythrocytes intensified platelet aggreability and mediated increased platelet recruitment. Erythrocytes were shown to induce a twofold increase in platelet thromboxane B2 (TXB2) synthesis upon collagen stimulation, indicating that erythrocytes actively modulated platelet eicosanoid formation and thereby activation (Valles J. et al., Blood, 1991, 78:154-162*).* A direct erythrocyte-platelet interaction was reported by Goel M.S. *et al.* (Goel M.S. et al., Blood, 2002, 100:3797-3803)*.* It was shown that normal erythrocytes could adhere to activated platelets under venous flow conditions. The erythrocyte-platelet adhesion was proposed to be a result of platelet glycoprotein (GP) VI (GPVI) signalling. Goel *et al.* also observed that this adhesion could be reduced by antibodies directed against CD36 or GPlb, but not by antibodies against GPIIb (anti-CD41a). However, the underlying mechanism and the physiological relevance of this direct erythrocyte-platelet contact remains to be identified.

Intercellular adhesion molecule 4 (ICAM-4 also designated Landsteiner-Wiener (LW) blood group glycoprotein or CD242) is an erythroid-specific membrane component that belongs to the family of immunoglobulin superfamily (IgSF) of proteins. ICAM-4 has been shown to preferentially but, not exclusively, interact with the beta part of several members of the integrin family such as LFA-1 (CD11a/CD18, α_{L}β₂) on leukocytes (Bailly P. et al., Eur. J. Immunol., 1995, 25:3316-3320), Mac-1 (CD11b/CD18, α_{M}β₂) on granulocyte/monocyte (Bailly P. et al., Eur. J. Immunol., 1995, 25:3316-3320), and monocyte/macrophage integrin CD11c/CD18 (α_{X}β₂) (Ihanus E. et al., Blood, 2007, 109:802-810)*.*

The platelet fibrinogen receptor α_{IIb}β₃ (platelet glycoprotein GPIIb-IIIa), which belongs to the cytoadhesin sub-class of the integrin family, is a receptor known to be responsible for platelet aggregation. It is noteworthy that α_{IIb}β₃ binds to its ligand only after platelet activation, after undergoing conformational changes (Plow, E. et al., Semin Thromb Haemostasis, 1992, 18, 324-332). It has also been demonstrated that ICAM-4 is a ligand for activated α_{IIb}β₃ integrin resulting in platelet-erythrocyte interaction. This interaction was shown to occur under *in vitro* static conditions, and to be inhibited by monoclonal antibodies specifically recognising the β₃-chain (CD61), α_{IIb}β₃ or ICAM-4 (Hermand P. et al., J. Biol. Chem., 2003, 278:4892-4898)*.*

The invention investigated the involvement of ICAM-4 and α_{IIb}β₃ in the direct erythrocyte-platelet interaction under near physiological conditions *in vitro.* The inventors also explored the physiological function of the ICAM-4 mediated erythrocyte-platelet interaction *in vivo.* As a result of their investigation, the inventors have identified that interaction between platelets and erythrocytes occurs through receptor/ligand interaction in physiological conditions or *in vivo,* wherein said ligand is ICAM-4 and said receptor is α_{IIb}β₃.

Based on their results obtained in an *in vitro* model of physiological conditions or *in vivo* on mice, the inventors have designed compounds and applications suitable for use in patients undergoing thrombotic or bleeding disorders.

Thus the invention relates to a polypeptidic compound capable of blocking ICAM-4 binding to platelet integrin α_{IIb}β₃ for use in the treatment of a thrombotic disease, when administered to an individual diagnosed with such a condition.

More particularly, the polypeptidic compound according to the invention is an anti-ICAM-4 antibody or a fragment thereof capable of blocking ICAM-4 binding to platelet integrin α_{IIb}β₃, or an ICAM-4 mimetic peptide capable of blocking ICAM-4 binding to platelet integrin α_{IIb}β₃, or an anti-CD61 antibody or a fragment thereof capable of blocking ICAM-4 binding to platelet integrin α_{IIb}β₃ for use in the treatment of a thrombotic disease, when administered to an individual diagnosed with such a condition.

As used herein, the expression *"for use in the treatment"* means that a compound as defined herein, in particular an anti-ICAM-4 antibody or a fragment thereof capable of blocking ICAM-4 binding to platelet integrin α_{IIb}β₃, or an ICAM-4 mimetic peptide capable of blocking ICAM-4 binding to platelet integrin α_{IIb}β₃, or an anti-CD61 antibody or a fragment thereof capable of blocking ICAM-4 binding to platelet integrin α_{IIb}β₃ can be used in a process of administration to an individual, in particular a human, to prevent or treat thrombotic diseases, by reducing or preventing thrombus formation, while limiting the risk of major bleeding. In a particular embodiment, treatment involving the anti-ICAM-4 antibody or a fragment thereof, or the ICAM-4 mimetic peptide, or the anti-CD61 antibody or a fragment thereof according to the invention may be accompanied by other therapies.

As defined herein, the expression *"fragment of an antibody"* refers to a portion of a full length antibody that retains the binding capacity of the full length antibody, *i.e*. by binding the antigen to which the full length antibody binds. Particular fragments according to the invention consist of variable fragments or heavy and/or light chains of antibodies, such as fragments encompassing all or part of CDR domains of heavy and/or light chains.

As defined herein, the expression *"ICAM-4 mimetic peptide"* refers to an ICAM-4 peptide mimicking the extracellular domain of ICAM-4, in particular said extracellular domain in human ICAM-4 such as the synthetic peptide Gly-Leu-Asp-Leu-Ala-Asn-Val-Thr-Leu-Thr-Tyr-Glu-Phe-Ala-Ala-Gly-Pro-Arg-Asp (GLDLANVTLTYEFAAGPRD, SEQ ID NO: 1), for its capacity to block ICAM-4 binding to platelet integrin α_{IIb}β₃. Human ICAM-4 is made up of 271 amino acids and its sequence is referenced under the protein accession number UniProtKB/Swiss-Prot: Q14773.1 (SEQ ID NO: 2).

As defined herein, the expression *"thrombotic disease"* refers to disorders of the haemostatic system that are commonly known to the person skilled in the art and that affect the haemostatic balance. In particular it encompasses venous and arterial thrombosis such as deep vein, portal, renal or jugular vein thrombosis, pulmonary embolism, Budd-Chiari syndrome, Paget-Schroetter disease, cerebral venous sinus thrombosis and myocardial infarction. More particularly, it refers to venous thrombosis such as deep vein thrombosis (DVT) and pulmonary embolism.

In a particular embodiment of the invention, said polypeptidic compound can be used for inhibiting erythrocyte-platelet adhesion.

In another particular embodiment of the invention, said polypeptidic compound can be used for inhibiting platelet deposition and fibrin formation at the site of injury.

According to a particular embodiment of the invention, said polypeptidic compound can be used for reducing fibrinogen binding to integrin α_{IIb}β₃ on platelets.

According to another particular embodiment of the invention, said anti-CD61 antibody or fragment thereof is used in combination with the ICAM-4 mimetic peptide, or with the anti-ICAM-4 antibody or fragment thereof according to the invention.

As defined herein, the expression *"used in combination"* means that said anti-CD61 antibody or fragment thereof, said ICAM-4 mimetic peptide, or said anti-ICAM-4 antibody or fragment thereof can be administered simultaneously or sequentially, in the same or separate pharmaceutical compositions.

In a particular embodiment of the invention, said anti-ICAM-4 antibody or fragment thereof is a polyclonal antibody directed against an epitope mapping an extracellular domain of ICAM-4.

In another particular embodiment of the invention, said anti-CD61 antibody or fragment thereof is a monoclonal antibody directed against the β-chain, in particular the β₃-chain of the platelet integrin α_{IIb}β₃.

In another particular embodiment of the invention, said ICAM-4 mimetic peptide corresponds to a synthetic peptide mimicking the extracellular domain of ICAM-4. More particularly, said ICAM-4 mimetic peptide is a synthetic peptide having the amino acid sequence Gly-Leu-Asp-Leu-Ala-Asn-Val-Thr-Leu-Thr-Tyr-Glu-Phe-Ala-Ala-Gly-Pro-Arg-Asp (GLDLANVTLTYEFAAGPRD) as disclosed in SEQ ID NO: 1. Said peptide is, as such, a particular embodiment of the invention.

The invention also relates to the use of a synthetic arginyl-glycyl-aspartic acid (RGD)-containing peptide to block ICAM-4 binding to platelet integrin α_{IIb}β₃.

The present invention also relates to a screening method to detect or monitor *in vitro* the effect of a substance, drug or pharmaceutical agent on the ICAM-4/α_{IIb}β₃ interaction in a biological sample, while simulating blood flow conditions existing *in vivo,* comprising the following steps:
a) mixing washed platelets and washed erythrocytes previously isolated from the biological sample,
b) pre-incubating the erythrocyte/platelet mixture with a platelet agonist for 2 minutes at 37°C,
c) carrying out the perfusion of the erythrocyte/platelet mixture on a platelet adhesive protein coated surface in a perfusion chamber under a venous flow shear rate for 5 minutes at 37°C,
d) acquiring images under a venous flow shear rate under differential interference contrast (DIC) microscopy,
e) quantifying the images to calculate the erythrocyte adherence.

In the present invention, the biological sample is selected from the group consisting of whole blood, plasma, or a derivative that would provide erythrocytes and platelets.

As defined herein, the expression *"while simulating blood flow conditions existing in vivo"* means that the perfusion is carried out under a low flow shear rate similar to the one observed in the blood vessel of an individual, in particular a human.

According to a particular embodiment of the invention, the flow shear rate used for perfusion is venous flow shear rate, within the range of 100 to 300 s⁻¹. The arterial flow shear rate is above 800 s⁻¹, and is usually not compatible with the erythrocyte-platelet interaction leading to thrombus formation *in vivo.* Under particular flow conditions, arterial thrombus can be targeted.

As defined herein, the term *"chamber"* refers to a vessel containing a fluid. In particular, the chamber according to the invention is a single-pass perfusion chamber, which contains an inlet, outlet, a vacuum channel, and on the top, a silicone rubber gasket.

As defined herein, the expression *"platelet agonist*" refers to a compound that induces platelet activation by binding to receptors on the platelet surface. Platelet agonists can be classified into two categories, strong agonists such as collagen, thrombin and thromboxane A2, and weak agonists such as ADP and epinephrine. In particular, thrombin is considered to be the most potent platelet activator *in vivo.* Unlike weak platelet agonists, strong platelet agonists can induce platelet granule secretion even when aggregation is inhibited.

According to a particular embodiment of the invention, the platelet agonist of step b) is selected from the group consisting of collagen-related peptide, thrombin, ADP and arachidonic acid.

As defined herein, the expression *"platelet adhesive protein"* refers to a protein that plays an essential role in primary hemostasis by promoting platelet adhesion. In the present invention, the platelet adhesive protein enables immobilisation of the platelets onto the coverslip.

According to a particular embodiment of the invention, the platelet adhesive protein is von Willebrand factor (VWF) or fibrinogen.

According to another particular embodiment of the invention, said substance, drug or pharmaceutical agent is added to the biological sample to be assayed or is present in the biological sample as a result of the patient being under treatment with this substance, drug or pharmaceutical agent.

The screening method of the invention can be used for detecting or monitoring a thrombotic disease.

According to a particular embodiment of the invention, said substance, drug or pharmaceutical agent blocks the ICAM-4/α_{IIb}β₃ interaction. Blocking of the interaction can be determined by detecting inhibition of platelet activation and accordingly measuring P-selectin expression as a marker of the activation level.

According to another particular embodiment of the invention, said substance, drug or pharmaceutical compound comprises a polypeptidic compound according to the invention, in particular an anti-ICAM-4 antibody or a fragment thereof, or an ICAM-4 mimetic peptide, or an anti-CD61 antibody or a fragment thereof according to the invention.

The invention also relates to a screening method for evaluating the risk of a patient to have a thrombotic condition, which comprises a step of measuring the expressed ICAM-4 in a biological sample previously obtained from a patient. In particular, this measurement can be carried out under ELISA-based methods.

According to a preferred embodiment of the invention, an abnormally high ICAM-4 level is a risk indicator for a thrombotic disease.

As defined herein, the expression *"abnormally high ICAM-4 level*" means that the ICAM-4 level is higher than 110% of normal ICAM-4 expression (arbitrary units).

The invention also relates to a screening method for evaluating the risk of a patient to have a bleeding phenotype, which comprises a step of measuring the expressed ICAM-4 in a biological sample previously obtained from a patient.

According to a preferred embodiment of the invention, an abnormally low ICAM-4 level is a risk indicator for a bleeding disease.

As defined herein, the expression *"abnormally low ICAM-4 level*" means that the ICAM-4 level is lower than 100% of normal ICAM-4 expression, in particular it falls between the ranges of 0-70% of normal ICAM-4 expression (arbitrary units).

The method of the invention thus further enables the determination of the minimal amount of polypeptidic compound according to the invention, in particular an anti-ICAM-4 antibody or fragment thereof, an ICAM-4 mimetic peptide, or an anti-CD61 antibody or a fragment thereof according to the invention, necessary to reduce or prevent thrombus formation, by blocking all or part of the ICAM-4/ α_{IIb}β₃ interaction.

According to a particular embodiment of the invention, the screening method can be used in combination with other screening tests.

According to another particular embodiment of the invention, the screening method enables the determination of the ICAM-4/α_{IIb}β₃ interaction by measuring thrombin generation.

The present invention is also directed to a pharmaceutical composition comprising an anti-ICAM-4 antibody or a fragment thereof, or an ICAM-4 mimetic peptide, or an anti-CD61 antibody or a fragment thereof as defined herein, as active ingredient, in association with a pharmaceutically acceptable vehicle, and optionally a distinct therapeutically active molecule for use in the treatment of a thrombotic disease, when administered to an individual diagnosed with such a condition.

As defined herein, pharmaceutically acceptable vehicles encompass any substance that enables the formulation of the anti-ICAM-4 antibody or ICAM-4 mimetic peptide according to the invention within a composition. Examples of pharmaceutically acceptable vehicles are well known to a person skilled in the art.

The present invention also concerns a kit for carrying out the above-defined screening method, which comprises:
- an anti-ICAM-4 antibody or a fragment thereof, or an ICAM-4 mimetic peptide, or an anti-CD61 antibody or a fragment thereof as defined herein; and
- a platelet agonist.

### BRIEF DESCRIPTION OF THE DRAWINGS

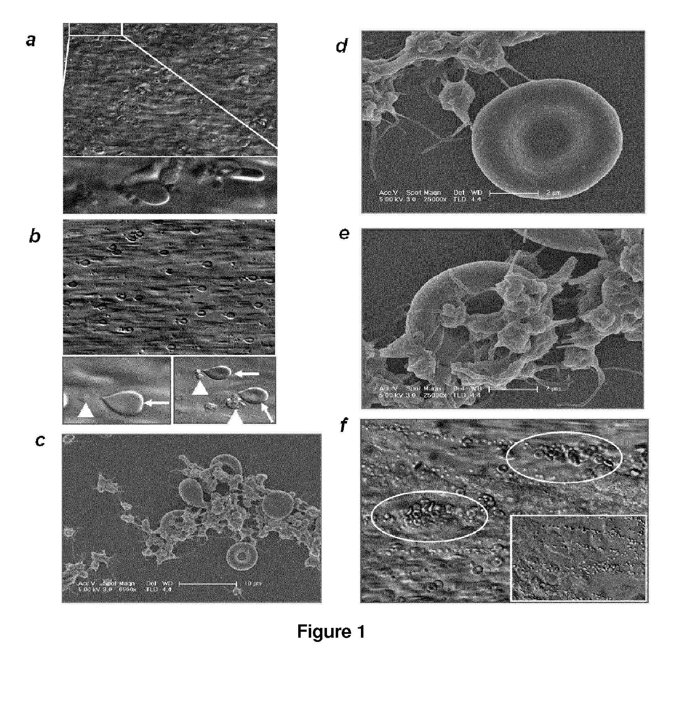

**Figure 1****. Erythrocyte binding to platelets under flow.** (a) Erythrocyte binding to platelets in whole blood. Citrated whole blood was perfused on collagen coated cover glass for 5 minutes at shear rate of 100 s⁻¹. (b) Erythrocyte binding to activated platelets in buffer. A mixture of isolated erythrocytes and platelets was perfused on von Willebrand factor (VWF) coated cover glass for 5 minutes at shear rate of 100 s⁻¹. Collagen related peptide (CRP) (500 ng/ml) was added into cell suspension before perfusion. White triangles point to platelets, and white arrows point to erythrocytes. (c-e) Scanning electronic microscope (SEM) image of erythrocyte binding to platelet aggregates. A mixture of isolated erythrocytes and platelets was perfused on VWF coated cover glass for 10 minutes at shear rate of 100 s⁻¹ in the presence of 500 ng/ml CRP. After perfusion, samples were fixed with 1% glutaraldehyde and applied for SEM analysis. (f) Erythrocyte involvement in platelet/erythrocyte aggregates following endothelial activation. Either platelet alone or erythrocyte/platelet mixture was perfused on endothelial cell coated cover glass for 10 minutes at 100 s-¹. Prior to perfusion, endothelial cells were activated with histamine. Image in rectangular box shows platelet decorated stings formed by platelet adhesion to ultra-large (UL) VWF secreted by activated endothelial cells. Area in white ellipse shows erythrocyte/platelet aggregates formed by erythrocyte/platelet mixture perfusion.

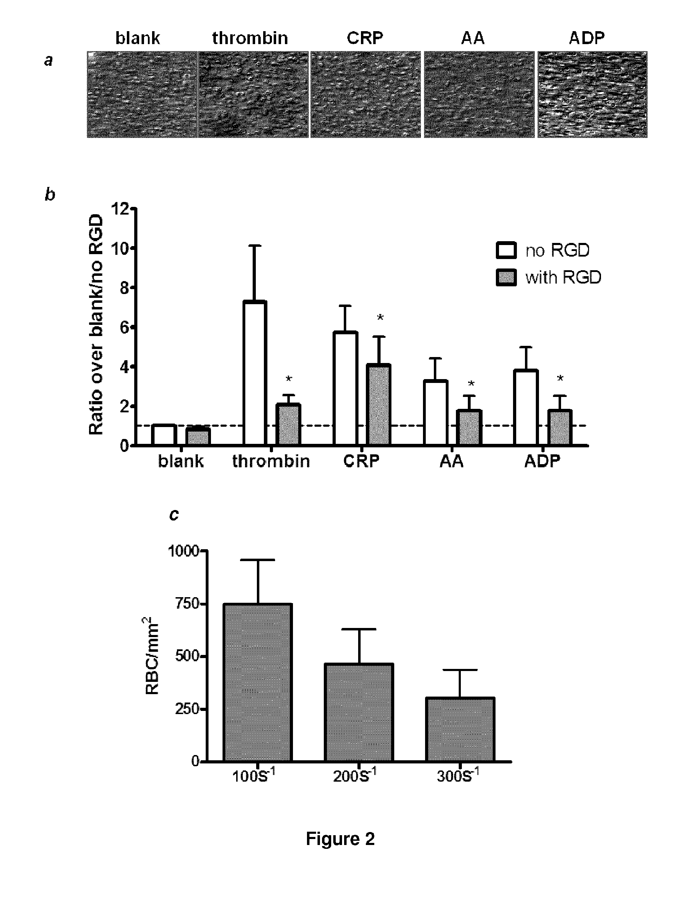

**Figure 2****. Influence of platelet activation status, RGD peptide and shear rate on erythrocyte binding to platelets under flow.** (a) Representative image of erythrocyte binding to platelets under different conditions. Erythrocyte/platelet mixture was perfused on VWF coated cover glass for 5 minutes at shear rate of 100 s⁻¹ in the presence of either blank or different platelet stimuli-thrombin (1U/ml), CRP (500 ng/ml), arachidonic acid (AA, 10 µM) and ADP (10 µM). (b) Erythrocyte binding to platelets in the presence of different stimuli and in the presence or absence of RGD peptide (100 µM) in the same settings as **Figure 1a****.** The results were expressed as mean ratio of bound RBC per mm² against non stimulated sample ± SEM of 4 experiments. By student *t* test analysis: * represents P<0.05 comparing RGD treated samples with untreated samples. (c) Influence of shear rate on erythrocyte binding to platelets. Erythrocyte/platelet mixture was perfused on VWF coated cover glass in the presence of CRP (500 ng/ml) for 5 minutes under indicated shear rate. Results were expressed as mean bound RBC per mm² ± SEM of 3 experiments.

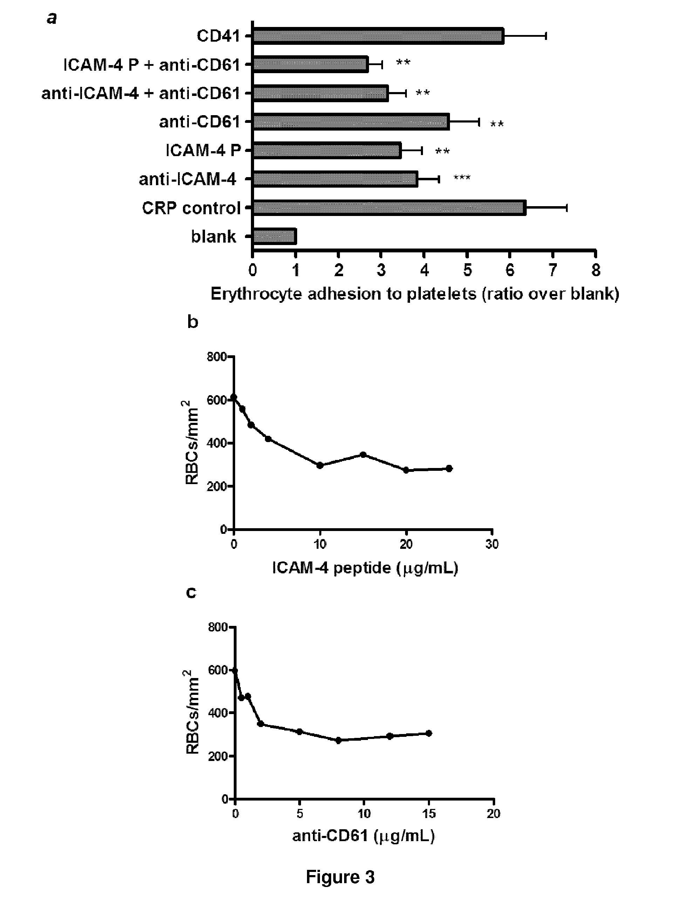

**Figure 3****. Influence of antibodies directed against α_{IIb}**β**₃ (anti-CD61 and anti-CD41), antibody against ICAM-4 (anti-ICAM-4) and ICAM-4 peptide on erythrocyte binding to activated platelets.** (a) Erythrocyte binding to activated platelets in the presence of indicated antibodies and peptide. Erythrocyte/platelet mixture was perfused on VWF coated cover glass for 5 minutes at shear rate 100 s⁻¹. Anti-CD61 (6.25 µg/ml), anti-ICAM-4 (10 µg/ml), ICAM-4 peptide (10 µg/ml) and combinations of anti-CD61 with anti-ICAM-4/ICAM-4 peptide were added into cell suspension and incubated at 37 °C for 3 minutes. CRP (500 ng/ml) was added into cell suspension prior to perfusion to activate platelets. The results were expressed as mean ratio of bound RBC per mm² against untreated sample (blank) ± SEM of 8 experiments. By student *t* test analysis: **, P<0.01; ***, P<0.001 comparing antibody or peptide treated samples to CRP control samples. (b-c) Dose depended inhibitory effect of ICAM-4 peptide and anti-CD61 on erythrocyte binding to activated platelets. ICAM-4 peptide or anti-CD61 were added into erythrocyte/platelet mixture at indicated concentrations and incubated at 37 °C for 3 minutes. CRP (500 ng/ml) was added into cell suspension prior to perfusion to activate platelets.

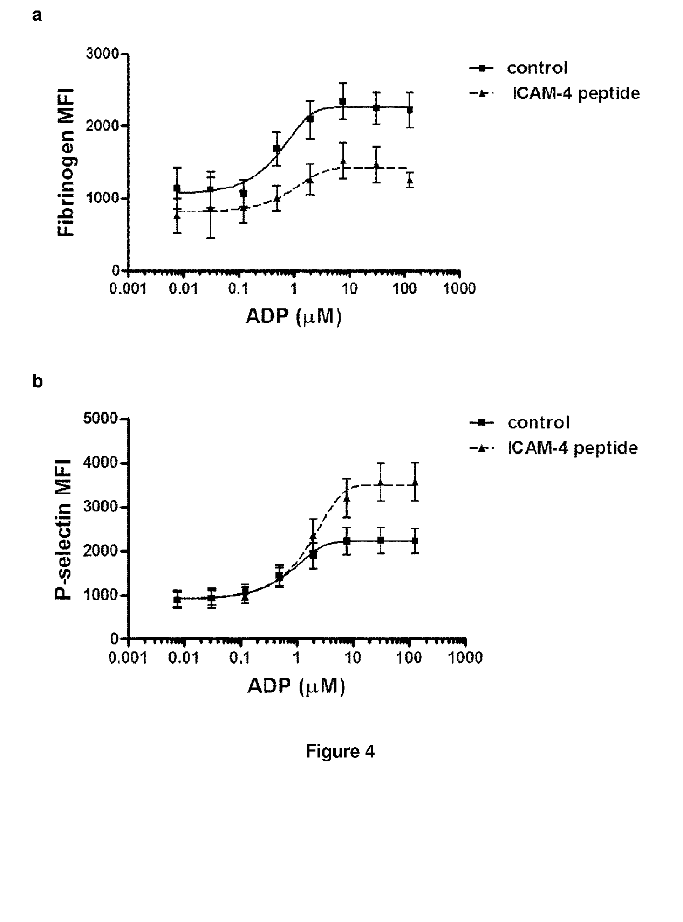

**Figure 4****. Effect of ICAM-4 peptide on (a) platelet fibrinogen binding and (b) P-selectin expression, under ADP stimulation.** Gradient concentrations of ADP and Alex Fluor 488-conjugated fibrinogen or R-phycoerythrin (RPE)-conjugated P-selectin were added into diluted whole blood sample (1:100 in PBS) in the presence or absence of the ICAM-4 peptide (10 µg/ml). The results were expressed as mean fluorescence intensity (MFI) ± SEM of 3 experiments.

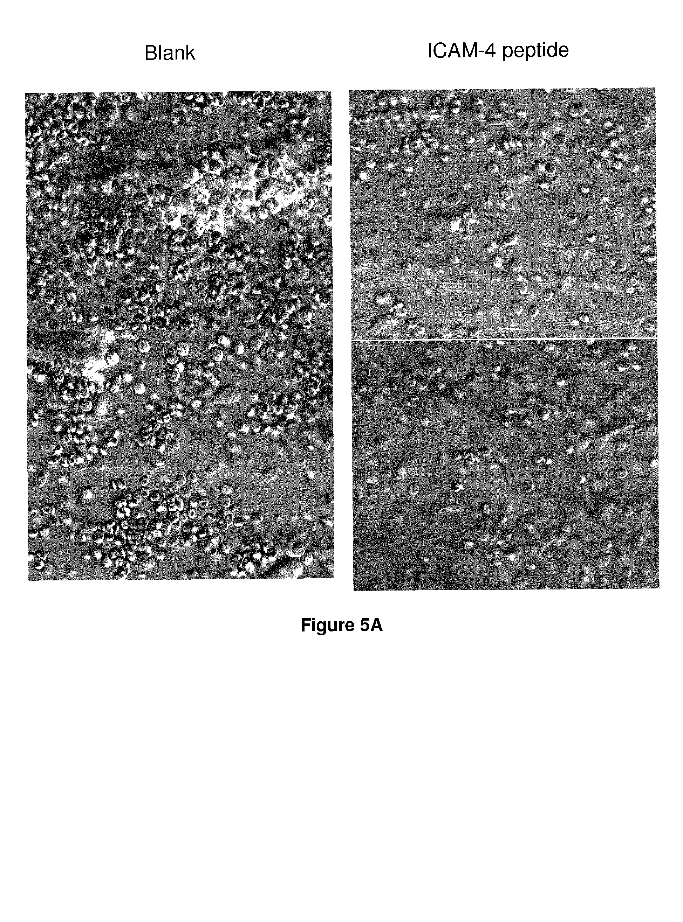

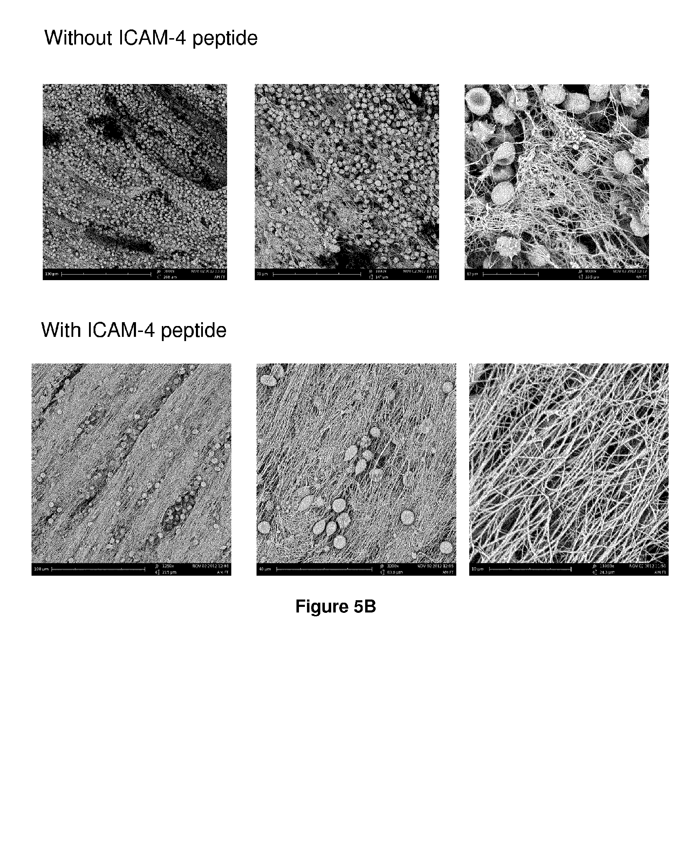

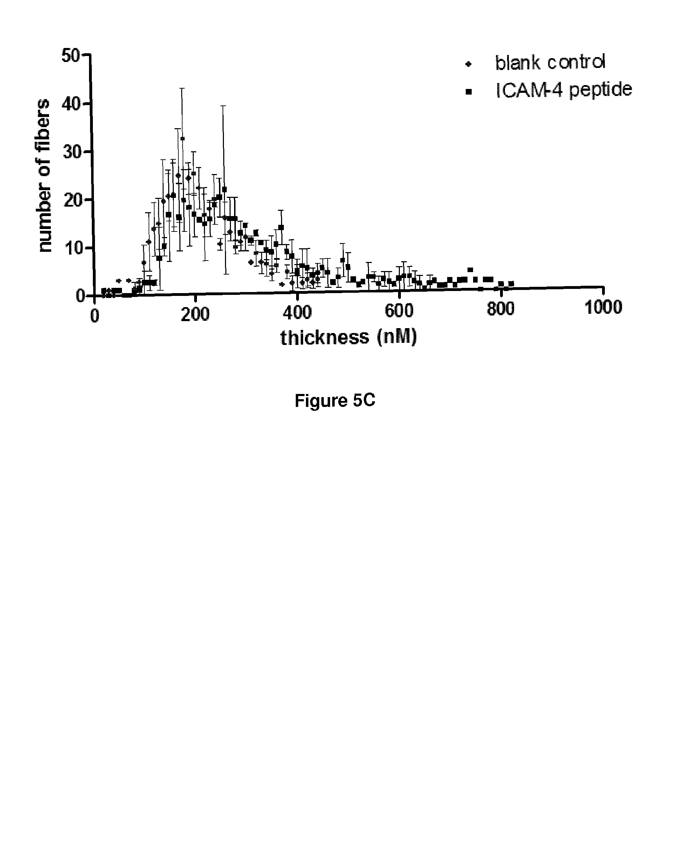

**Figure 5****. Effect of ICAM-4 peptide on fibrin formation.** Fluorescent labelling of fibrinogen and platelets was carried out. Perfusion experiments were performed with isolated labelled platelets and unlabelled erythrocytes. This cell suspension was perfused over a VWF-coated surface in the presence of CRP and fluorescently labelled fibrinogen **(****Figure 5A****).** **Figure 5B** shows SEM pictures of the above-described experiments. **Figure 5C** displays fibre thickness differences between the two conditions.

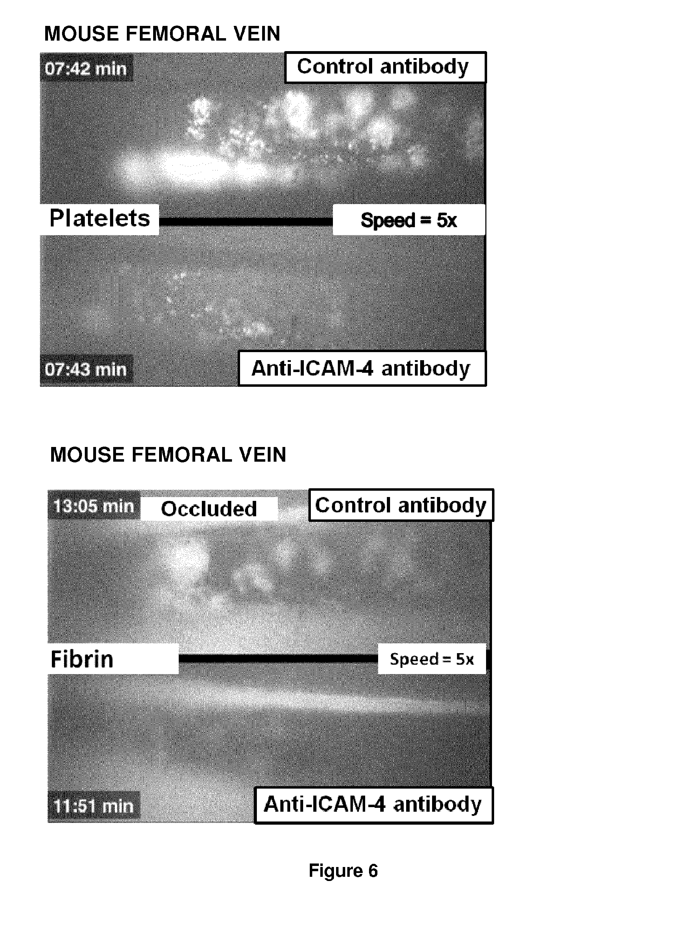

**Figure 6****. Effect of anti-ICAM-4 antibody on experimental thrombosis in mice (*in vivo* experiments).** Ferric chloride (4.5%, 0.4 µL drop for 1 minute) was applied topically to the femoral vein of C57BL/6J wild type mice to induce vascular injury. Ten minutes before injury, the mouse was treated either with a polyclonal rabbit anti-mouse ICAM-4 antibody (0.7 µg/g body weight) or with a control antibody (0.7 µg/g body weight). Platelet deposition was visualised using calcein-red-orange-labelled donor platelets (2x10⁶/g body weight) and fibrin formation was visualised using a FITC-labelled anti-fibrin antibody (0.8 µg/g body weight). Indicated time represents the time after injury.

### EXAMPLES

### Example 1

### Materials

**Antibodies and proteins.** Antibodies: R-phycoerythrin (RPE) labeled mouse anti-human P-selectin antibody, mouse anti-human CD61 (integrin β3 chain) monoclonal antibody, mouse anti-human CD41a (integrin α2b chain) monoclonal antibody were purchased from BD biosciences (Franklin Lakes, NJ, U.S.A). Goat anti-human ICAM-4 (epitope mapping within an extracellular domain of ICAM-4) polyclonal antibody and ICAM-4 mimetic peptide (corresponding to the anti-ICAM-4 epitope) were purchased from Santa Cruz biotechnology inc (Delaware Avenue, CA, USA). Polyclonal rabbit anti-mouse ICAM-4 was purchased from Santa Cruz Biotechnology, Dallas, TX USA. Proteins/peptides: Human von Willebrand factor (VWF) was purified as described by Huizinga *et al.* (Huizinga E.G. et al., Science, 2002, 297:1176-1179)*.* Human fibrinogen (plasminogen, VWF and fibronectin free) were obtained from Enzyme Research Labs (South Bend, IN, USA). Thrombin was purchased from Enzyme Research Laboratories (South Bend, IN). Adenosine diphosphate (ADP) was purchased from Roche (Almere, The Netherlands). Cross-linked Collagen Related Peptide (CRP) was synthesised by R. Farndale as previously described *(*Slatter et al., Peptides, 2012, 36, 86-93). Alex Fluor 488 conjugated fibrinogen was purchased from Molecular Probes Inc (Eugene, OR, U.S.A). The RGD containing peptide D-Arginyl-Glycyl-L-Aspartyl-L-Tryptophan (dRGDW) was synthesised at the department of Membrane Enzymology, Faculty of Chemistry, University of Utrecht, the Netherlands. Arachidonic acid was bought from Bio/data Corporation (Horsham, PA, U.S.A). Collagen Type I (Kollagenreagen Horm Suspension) was purchased from NYCOMED (Austria GmbH, Linz, Austria).

**Cell isolation.** Blood from healthy volunteers was drawn into one-tenth volume of 3.2% sodium citrate. All healthy volunteers claimed not to have used aspirin or other nonsteroidal anti-inflammatory drugs for the preceding 10 days. Washed platelets were prepared as previously described by Lisman *et al.* (Lisman T. et al., Blood, 2003, 101:1864-1870). Briefly, blood was centrifuged at 156g for 15 min at room temperature. The platelet-rich plasma (PRP) was removed and acidified by addition of one-tenth volume of ACD (2.5% trisodium citrate, 1.5% citric acid, and 2% D-glucose). Platelets were centrifuged (330g, 15 min) and the platelet pellet was resuspended in Hepes-Tyrode buffer at pH 6.5 (10 mM Hepes [N-2-hydroxyethylpiperazine-N'-2ethanesulfonic acid], 137 mM NaCl, 2.68 mM KCI, 0.42 mM NaH₂PO₄, 1.7 mM MgSO₄, 5 mM D-glucose). Prostacyclin (PGI₂, 10 ng/ml) was added to prevent platelet activation during the subsequent washing step. Platelet suspension was centrifuged (330g, 15 min) and resuspended in a small volume of HEPES-Tyrode buffer at pH 7.3.

Erythrocytes were isolated from the same blood sample as washed platelets by using PEGG elution column and cellulose (Beutler E. et al., Blood Cells, 1986, 12:57-64). Briefly, α-cellulose and cellulose type 50 were suspended in saline (0.9% NaCI) at ratio of 1:1 and were consecutively poured into elution column. After saline was drained from the column, 3 to 5 ml whole blood was added onto the top of the cellulose. Column was placed in a clean 50 ml tube and centrifuged at 50g at room temperature for 5 min. 5 ml saline was added to the top of the cellulose and was centrifuged again at 50g for 5 min. Column was removed from the 50 ml tube. 50 ml tube with eluted erythrocytes was filled with saline and centrifuged at 1000g for 5 min at room temperature. Erythrocytes were washed again with Ringer solution (32 mM HEPES, 125 mM NaCl, 5 mM KCI, 1 mM MgSO₄, 5 mM glucose, 1 mM CaCl₂, pH=7.4) and finally resuspended in Ringer solution.

### Perfusion experiments

An *in vitro* perfusion system connected to a light microscope and a digital camera was used to study erythrocyte binding to platelets adhered to surfaces coated with different adhesive proteins at different flow rates.

*Sample preparation.* Washed platelets and washed erythrocytes were mixed in ringer buffer to reach platelet count of 150 x 10⁹/L and hematocrit of 5%. Mixture of platelets and erythrocytes was kept at 37 °C before and during perfusion.

*Perfusion on a platelet adhesive protein coated surface and image quantification.* Glass coverslips (24×50 mm, Menzel Gläser) were precleaned overnight with chromosulfuric acid (2% chromium trioxide) solution and rinsed with distilled water before coating. Platelet adhesive proteins (VWF or fibrinogen) were immobilised on coverslips by 1.5 hour incubation with 300 µl at 30 µg/ml VWF or 300 µl at 100 µg/ml fibrinogen. After coating, the coverslips were blocked by 1.5% human albumin in phosphate-buffered saline (PBS; 10 mmol/L sodium phosphate, 150 mmol/L NaCl, pH 7.4) for at least one hour at room temperature.

Perfusions were carried out in a single-pass perfusion chamber as previously described by Sixma *et al.* (Sixma J.J. et al., Thromb. Res., 1998, 92:S43-S46)*.* The chamber contained an inlet, outlet, a vacuum channel, and on the top, a silicone rubber gasket. A flow hole was bored in this silicone gasket to create a perfusion slit (slit width: 2 mm; length: 3 cm; height = thickness of sheet: 0.125 mm) connecting the inlet with the outlet. On both sides, a slit was cut in the silicon gasket next to the flow area to obtain two vacuum compartments. The coverslip was mounted on this silicone sheet by a vacuum force through the vacuum channel. The chamber together with protein coated coverslip was put upside down on an inverted optical microscope (Carl Zeiss Axio observer. Z1, Oberkochen, Germany) with a CCD camera attached. Samples of erythrocyte/platelet mixture or whole blood were pre-warmed to 37 °C and was drawn for 5 min through the perfusion chamber by a syringe placed in an Harvard infusion pump (pump 22, model 2400-004; Natick, MA, USA) by which different wall shear rates were maintained. After 5 minutes of perfusion, more than 10 pictures were taken under flow at different field views under differential interference contrast (DIC) setting. In some experiments, erythrocyte/platelet mixture was preincubated with CRP (500 ng/ml), thrombin (1 Unit/ml), ADP (10 µM), arachidonic acid (AA, 10 µM), dRGDW (100 µM), anti-GP1b AK2 (4 µg/ml), anti-CD61 (6.25 µg/ml), anti-CD41 (10 µg/ml), anti-ICAM-4 (10 µg/ml), or ICAM-4 peptide (10 ug/ml) for 2 min before perfusion. Images were quantified by counting erythrocyte number on each picture by ImageJ software. The erythrocyte adherence per mm² area was calculated and normalised against control.

**Scanning electron microscopy.** Two types of Scanning Electron microscopes were used. A tabletop model (PhenomWorld, Phenom Desktop SEM PRO) for quick scanning and a high end model (Phlips XL 30) for in-depth visualisation. For both types of SEM's, samples were treated in the same way. After perfusion of erythrocyte/platelet mixture samples or recalcified whole samples on VWF or collagen coated surfaces for 5 or 10 minutes, samples were directly fixed by perfusion of 1% glutaraldehyde/PBS for another 10 min at the same shear rate. After fixation, samples were treated with serial concentrations of ethanol and kept in 96% ethanol until SEM analysis. The samples were sputter-coated with a thin layer of 6.5 nm platinum or 20 nm gold in a sputter coater. The inventors used an automated algorithm named Fibermetric from Phenom World (Eindhoven, Netherlands) to calculate the thickness of the fibrin fibres and the pore size between the fibrin fibres.

**Fibrinogen binding and P-selectin expression assay by flow cytometry.** Fibrinogen binding and P-selectin expression on platelets related to activation with different agonists in the absence or presence of ICAM-4 peptide (10 µg/ml) were determined with concentration series of ADP and CRP. Serial dilutions of ADP (125 µM, 31 µM, 8 µM, 2 µM, 490 nM, 120 nM, 31 nM, 8 nM) were prepared in 50 µL HEPES buffered saline (HBS;10 mM HEPES, 150 mM NaCl, 1 mM MgS04, 5 mM KCI, pH 7.4) with 2 µL PE labeled mouse anti-human P-selectin antibody and 0.5 µL Alex Fluor 488 conjugated fibrinogen. PE conjugated mouse IgG1 was used as isotype control for P-selectin staining. 5 µL fresh, citrate anticoagulated whole blood was added to each sample of the above serial dilutions and to isotype control sample. After 20 minutes of incubation, the samples were fixed with 500 µL of 0.2% formyl saline (0.2% formaldehyde in 0.9% NaCl). All samples were analysed on a FACSCalibur flow cytometer from BD Biosciences (Franklin Lakes, NJ, U.S.A.) at the same day of processing. Single platelets were gated based on forward and side scatter properties. The mean fluorescence intensity (MFI) in PE and FITC (for Alex Fluor 488 measurement) channels in the platelet gate was used as output for platelet P-selectin expression and fibrinogen binding respectively.

### Results

### Erythrocyte binding to platelets under flow.

To study erythrocyte-platelet interactions, the inventors performed *in vitro* flow experiments. The inventors found that erythrocytes bound to platelets both in buffer and in whole blood under a low shear flow. In particular, whole blood was perfused over collagen at different shear rates (100-300 s⁻¹) all within the venous shear rate range. Platelets were captured from blood stream and adhered on collagen during perfusion. About one minute later, erythrocytes attached to platelets with a sort of "focal adhesion point", resulting in a tear-drop shape **(****Figure 1A****).** The duration of the direct contact between erythrocyte and platelets under the low shear flow varies between a few seconds to a few minutes. In addition, this adhesion was inversely correlated with flow shear rate and predominately occurred at shear rates lower than 300 s⁻¹ **(****Figure 2C****).** Perfusion of isolated platelets and erythrocytes in physiological buffer on VWF coated surface in the presence of collagen related peptides (CRP) gave the same pattern of erythrocyte binding to platelets **(****Figure 1B****).** Scanning electron microscopy analysis showed erythrocytes binding to and integrated in platelet aggregates **(****Figures 1C-1E). Figure 1** **F** displays the binding of erythrocytes to platelets that adhere to endothelial derived von Willebrand Factor.

### Identification of platelet receptor responsible for binding erythrocytes.

To study the erythrocyte-platelet interactions in more detail, the inventors performed flow experiments with potential blocking agents to receptors on both platelets and erythrocytes.

Various platelet agonists (Thrombin 1 U/mL, CRP 500 ng/mL, AA 10 µM and ADP 10 µM) were added to a mixture of erythrocytes and platelets before perfusion. Subsequently the samples were perfused over a VWF coated surface for 5 minutes at a shear rate of 100 s⁻¹. As expected, the inventors observed increased erythrocyte binding to platelets in the presence of the different platelet agonists (3.2 to 7.1 fold increase depending on the agonist (exact fold increase), **Figures 2A and 2B****),** indicating that platelets in an increased activation state were more capable of capturing erythrocytes from the blood flow. Since the conformational change of platelet integrin α_{IIb}β₃ from an inactive form to an active form (fibrinogen binding form) upon stimulation was the pivotal step in platelet activation and aggregation (Bennett J. S. et al., J. Clin. Invest., 2005, 115:3363-3369)*,* the inventors investigated the involvement of this integrin in erythrocyte-platelet contact. An Arg-Gly-Asp (RGD) containing peptide (d-RGDW) known to inhibit platelet aggregation by binding to α_{IIb}β₃ was added to a mixture of erythrocytes and platelets in the presence of platelet agonists (Saelman E.U. et al., Arterioscler. Thromb., 1993, 13:1164-1170)*.* Samples were then perfused on VWF surface for 5 min. The inventors showed that RGD peptide inhibited erythrocyte binding to platelets at all agonist conditions (29% to 72% decrease, depending on the agonist used, p<0.05, n=4) **(****Figure 2B****),** indicating the involvement of α_{IIb}β₃ in erythrocyte-platelet contact.

### Erythrocyte-platelet interaction under flow was ICAM-4 and integrin α_{IIb}β₃ dependent.

As ICAM-4 on erythrocytes has been reported to be a ligand for α_{IIb}β₃ on platelets (Hermand P. et al., Eur. J. Biochem., 2004, 271:3729-3740*;* Hermand P. et al., J. Biol. Chem., 2003, 278:4892-4898)*,* the inventors tested whether this ICAM-4-α_{IIb}β₃ interaction was responsible for the erythrocyte-platelet adhesion. Inhibitory antibodies (anti-ICAM-4, anti-CD61) and an ICAM-4 peptide mimicking the extracellular domain of human ICAM-4 were added to separate erythrocyte-platelet mixture samples together with CRP before perfusion. Samples were perfused over VWF surface for 5 minutes. The inventors observed that the erythrocyte-platelet adhesion under conditions of flow was inhibited with both anti-ICAM-4 (40%, p<0.01, n=8) and anti-integrin β₃ (CD61) (46%, p<0.001, n=8). In addition, ICAM-4 peptide also generated a significant inhibitory effect on erythrocyte-platelet adhesion **(****Figure 3A****).** Both anti-CD61 and the ICAM-4 peptide showed a dose dependent inhibitory effect **(****Figures 3B and 3C****)** on erythrocyte binding to platelets.

In order to further explore the downstream events of ICAM-4 binding to integrin α_{IIb}β₃, the inventors studied the effect of the ICAM-4 peptide on platelet fibrinogen binding and P-selectin expression. Thus, gradient concentrations of ADP, and Alex Fluor 488-conjugated fibrinogen or RPE-conjugated P-selectin were added into diluted whole blood samples, in the presence or absence of the ICAM-4 peptide. Fibrinogen binding and P-selectin expression was measured on platelet surface by FACS. Comparing to the blank control, the inventors found a decreased fibrinogen binding to platelets (43% for ADP concentration at 125 µM, p<0.05, n=5) and an increased P-selectin expression (60%, p<0.01, n=5) on platelets upon ADP stimulation in the presence of ICAM-4 peptide **(****Figures 4A and 4B****).** Thus, the ICAM-4 peptide mimicking an extracellular domain of ICAM-4 promoted platelet P-selectin expression and blocked fibrinogen binding to integrin α_{IIb}β₃ on platelets. These results suggested that the ICAM-4 mimetic peptide competed with fibrinogen for binding to activated α_{IIb}β₃. Moreover, the increase of P-selectin expression in the presence of the ICAM-4 peptide suggested that the binding of ICAM-4 peptide to α_{IIb}β₃ resulted in outside-in signalling and further platelet activation.

### Blockage of the erythrocyte-platelet interaction led to reduced fibrin formation under a flow-based perfusion experiment.

The previously described results indicated that platelets were less able to bind fibrinogen in the presence of the inhibitory ICAM-4 peptide. To analyse whether this affects fibrin network formation, fluorescent labelling of fibrinogen and platelets was carried out. Perfusion experiments were performed with isolated labelled platelets and unlabelled erythrocytes. This cell suspension was perfused over a VWF-coated surface in the presence of CRP and fluorescently labelled fibrinogen. As evident from **Figure 5A****,** control samples demonstrated high numbers of platelets bound to the coated surface and a dense homogeneous fibrin network. In contrast, addition of ICAM-4 peptide in this system resulted in lower amounts of bound platelets and a less dense fibrin network concentrated around the bound platelets **(****Figure 5A****).** SEM analysis of these samples showed lower amounts of erythrocytes binding to the cover glass when ICAM-4 peptide was added as compared to the control **(****Figure 5B****),** confirming the results depicted in **Figure 3A****.** When analysing the fibrin network by SEM, blockage of ICAM-4 resulted in a less dense network with thicker fibres as compared to control **(****Figure 5C****).** These results indicated that red blood cells were necessary to obtain a suitable microenvironment for optimal fibrin network formation.

*In vivo* inhibitory effect of anti-ICAM-4 antibody in a mice thrombosis model.

A FeCl₃-based experimental thrombosis model in mice was applied to study the effect of an anti-ICAM-4 antibody according to the invention on thrombus formation. Platelet deposition and fibrin formation was recorded under a fluorescence microscope with calcein-labelled donor platelets and FITC-labelled anti-fibrin antibody respectively **(****Figure 6****).** The *in vivo* thrombosis model in mice demonstrated that the anti-ICAM-4 antibody profoundly inhibited platelet deposition and fibrin formation at the site of injury, thus preventing thrombus formation. Comparing to control mice, which showed a vessel occlusion time of approximately 10 minutes, the anti-ICAM-4 antibody treated mice showed no vessel occlusion in the first 20 minutes.

Using an *in vitro* perfusion system, the inventors demonstrated a direct erythrocyte-platelet adhesion under venous flow shear rate. The adhesion of erythrocytes to platelets was dependent on the structural opening up of platelet integrin α_{IIb}β₃. An antibody against the β₃ chain (anti-CD61) but not against the α_{IIb} chain (anti-CD41) of the integrin inhibited the erythrocyte-platelet adhesion. An ICAM-4 mimetic peptide and an anti-ICAM-4 demonstrated similar inhibitory effect under flow.

Application of the ICAM-4 mimetic peptide to platelets treated with serial concentrations of ADP led to higher platelet reactivity, demonstrated by higher P-selectin expression, implying that the binding of the ICAM-4 peptide to activated platelets induced outside-in signalling in platelets.

The blockage of the interaction through ICAM-4/integrin α_{IIb}β₃ led to reduced fibrin formation *in vitro* and reduced thrombus formation *in vivo.* These data indicated an active role of erythrocytes in thrombosis and haemostasis through interaction with activated platelets.

The interaction between erythrocytes and platelets has been reported by Santos *et al.* (Santos M. T. et al., J. Clin. Invest. 1991, 87:571-580*;* Valles J. et al., Blood, 1991, 78:154-162*;* Valles J. et al., Blood, 2002, 99:3978-3984). Using a two-stage *in vitro* system, Santos *et al.* showed that metabolically active erythrocytes could amplify platelet activation (demonstrated by increased release of platelet α-granule proteins and β-thromboglobulin, and increased synthesis of TXA) and that the cell-free releasates from platelets in the presence of erythrocytes had greater recruitment capacity than those from stimulated platelets alone (Santos M. T. et al., J. Clin. Invest., 1991, 87:571-580*;* Valles J. et al., Blood 1991, 78:154-162)*.* The components of platelet releasates were hypothesised to act as extracellular mediators between erythrocytes and platelets that brought about biochemical modifications by generating prothrombotic activity on erythrocytes.

In addition to this model, the present invention proposes a receptor/ligand dependent interaction between platelets and erythrocytes at a close to physiological experimental setting. The fact that an ICAM-4 mimetic peptide could induce a higher P-selectin expression on platelets implied that the direct erythrocyte binding to platelets might cause an outside-in signalling in platelets and thus further promote platelet activation. A high P-selectin expression together with enhanced α_{IIb}β₃ activation was also observed by the research group of Santos in their chemical messenger model of the erythrocyte-platelet interaction (Valles J. et al., Blood, 1991, 78:154-162)*.* Interestingly, the inventors found that both the chemical messenger model of Santos and the receptor/ligand model of the invention supported a promoting effect of the erythrocyte-platelet interaction on platelet activation, and that this effect was platelet stimulus dependent. It was unlikely that these two models were involved in two independent mechanisms in thrombosis. Thus, the inventors hypothesised that in the situations of platelet activation (provided a low flow environment, which might happen in venous system, or the downstream side of a platelet thrombi), erythrocytes would bind to platelets *via* ICAM-4/integrin α_{IIb}β₃. The interaction through ICAM-4/integrin α_{IIb}β₃ would lead, on the platelet side, to outside-in signalling and further activation of platelets, while on the erythrocyte side, it might cause erythrocyte cell deformation (through shear influence and probably erythrocyte cytoskeleton reformation), followed by a possible signalling in erythrocytes and a biochemical messenger release.

Although the net effect of erythrocyte-platelet interactions on platelets was positively regulated, the erythrocyte-platelet interaction under physiological conditions might also possess negative regulatory machinery. Several studies have demonstrated that ATP release from erythrocytes could be triggered by both mechanotransduction pathway (in response to shear stress and/or cell deformation) and receptor mediated pathways (in response to ADP) (Wan J. et al., Integr. Biol. (Camb.), 2011, 3:972-981*;* Knofler R. et al., Am. J. Hematol., 1997, 56:259-265)*.* ATP, AMP and adenosine are well-known inhibitors of the ADP induced stimulation of platelets (Macfarlane D.E. et al., Blood, 1975, 46:309-320)*.* ATP acts as a competitive inhibitor of ADP on the receptors and can stimulate the release of nitroxide and prostacyclin from endothelial cells.

The impact of direct erythrocyte-platelet interaction on thrombus formation and thrombin (fibrin) generation is difficult to explain solely by its positive effect on platelet activation. In the invention, the effects of the ICAM-4 mimetic peptide were seemingly paradoxical. Application of this peptide to activated platelets led to further platelet activation, which would predict a higher thrombotic status. Yet application of the same peptide in the *in vitro* perfusion model and the animal thrombosis model revealed a profound inhibitory effect of this peptide on thrombus formation/fibrin formation.

Considering that the anti-ICAM-4 antibody demonstrated similar inhibitory effect in the *in vivo* model, the inventors proposed that the inhibitory effect was achieved *via* blocking the interaction between ICAM-4 and the integrin α_{IIb}β₃. Combined with the fact that the anti-thrombotic tendency induced by ICAM-4/integrin α_{IIb}β₃ blockage through ICAM-4 peptide outweight the pro-thrombotic tendency induced by the binding of the peptide (involving intact erythrocyte) to platelets, the inventors thought that erythrocyte-platelet interaction carried out its pro-thrombotic influence *via* modifying erythrocyte biochemical properties. Recently, both research groups from Mann, K.G. and Hemker, H.C. have independently demonstrated a profound contribution of erythrocytes to thrombin generation (TG) using two different experimental approaches (Whelihan M.F. et al., Blood, 2012, 120:3837-3845*;* Ninivaggi M. et al., Clin. Chem., 2012, 58:1252-1259)*.* Erythrocytes participated in thrombin generation in tissue factor-activated blood, producing a membrane that supported prothrombin activation through the meizothrombin pathway. Surprisingly, erythrocytes contributed more than platelets to the procoagulant blood cell membranes necessary for optimal TG. Very interestingly, the ability of erythrocytes to directly activate coagulation was demonstrated by Iwata, H. *et al.,* who showed that erythrocytes had an elastase like enzyme that could activate FIX (Iwata H. et al., Biochem. Biophys. Res. Commun., 2004, 316:65-70)*.* The erythrocyte-platelet adhesion was probably the first step to introduce the erythrocyte as the procoagulant blood cell membrane. Since erythrocytes are known to have signal transduction pathways (Minetti G. et al., Curr. Opin. Hematol., 1997, 4:116-121)*,* the inventors hypothesised that erythrocyte-platelet adhesion could induce signalling transduction inside erythrocytes. However this hypothesis needed further investigation.

The inventors demonstrated that an Arg-Gly-Asp (RGD) containing peptide (d-RGDW), known to inhibit α_{IIb}β₃ mediated platelet aggregation, inhibited erythrocyte-platelet adhesion with 29% to 72%, depending on the agonist used (p<0.05, n=4). RGD peptides demonstrated the greatest inhibitory effect on erythrocyte binding to platelets in thrombin stimulated samples (72% decrease in erythrocyte binding), and the least inhibitory effect in CRP stimulated samples (29%). On the one hand, this difference in RGD inhibitory effect could be caused by the differences in the stimulation potencies of the agonists; on the other hand, it might be generated from the difference in the activation pathways induced by these agonists. It has been reported that thrombin, ADP and AA bind to various receptors on platelets and lead to a common pathway involving α_{IIb}β₃ conformational change (Angiolillo D.J. et al., Circ. J., 2010, 74:597-607)*.* Interestingly, collagen is known to activate not only the α_{IIb}β₃ but also the α_{IIb}β₁ on platelets (Nieswandt B. et al., Blood, 2003, 102:449-461)*.* It has also been shown that the RGD peptide is able to inhibit the function of α_{IIb}β₃ but not the one of α_{IIb}β₁ *(*Eble et al., 2001, J. Biol. Chem., 276:12274-84). Therefore, the inventors suspected that the reduction in the inhibitory effect of the RGD peptide on CRP treated samples might indicate that the integrin α_{IIb}β₁ might be a candidate for erythrocyte-platelet interaction in addition to α_{IIb}β₃. However the interaction between α_{IIb}β₁ and ICAM-4 has not been described in the literature and thus it needs further biochemical study to confirm this hypothesis.

ICAM-4-αᵥβ₃ interaction is known to be responsible for sickle cell adhesion to endothelium and consequently to lead to vaso-occlusion in the microcirculation (Zennadi R. et al., Blood, 2004, 104:3774-3781). Epinephrine was found to activate the adhesion of sickle cells, but not the one of normal red cells in a protein kinase A dependent fashion. It has been demonstrated that, unlike ICAM-4 on normal erythrocytes, ICAM-4 on sickle cells is phosphorylated (Zennadi R. et al., Blood, 2004, 104:3774-3781). Furthermore, ICAM-4 peptides have been synthesised to block this interaction as a treatment for sickle cell disease (Kaul D.K. et al., Am. J. Physiol Cell Physiol., 2006, 291:C922-C930). Mouse and human ICAM-4 protein have marked similarities with 68% overall identity. Critical cysteine residues and other key residues within the two extracellular IgSF domains are conserved, suggesting that these disulfide-bonded domains are similarly folded in human and murine proteins and may have analogous functional properties (Lee G. et al., Blood, 2003, 101:1790-1797). Targeted gene deletion of murine ICAM-4 shows that deficient mice live and do not show any gross abnormalities (Lee G. et al., Blood, 2006, 108:2064-2071)*.* Thus their hemoglobin content, hematocrit value and other red cell indices were normal. However the number of erythroblastic islands decreased to about half of that of normal animals. These islands are formed by a central macrophage surrounded by immature erythroid cells. Evidently, the decreased adhesion of the immature red cells to the macrophages (*via* ICAM-4-CD11c/CD18 interaction) results in the lower formation of islands. In the present invention, the inventors demonstrated a potential biological function of ICAM-4 through its interaction with platelet α_{IIb}β₃. Blockage of ICAM-4-α_{IIb}β₃ interaction led to reduced platelet activation, thrombin formation and fibrin deposition in an experimental thrombosis model. Application of the experimental thrombosis model in ICAM-4 knock-out mice would further elucidate the function of ICAM-4 in thrombosis. The inventors hypothesised that thrombus formation in knock-out mice would be less vigorous comparing to normal mice. The lack of gross abnormalities in the ICAM-4 gene deletion study carried out by Lee et al. (Lee G. et al., Blood, 2006, 108:2064-2071) suggested that ICAM-4 played a facilitating rather than an essential role in haemostasis. Thus, blocking ICAM-4 function would reduce thrombosis and yet preserves haemostasis. This would make ICAM-4 an ideal targeting medicine for thrombosis treatment. ICAM-4 targeting therapeutic approaches (*e.g*. ICAM-4 antibodies and/or ICAM-4 mimetic peptides) may hold promise to reduce morbidity and mortality associated with thrombosis.

In conclusion, the inventors observed a direct erythrocyte-platelet interaction under conditions of low shear. This interaction was partly mediated *via* erythrocyte-receptor ICAM-4 and α_{IIb}β₃ on platelets. In addition, the inventors found that disruption of the ICAM-4 mediated erythrocyte-platelet interaction *in vivo* by an anti-ICAM-4 antibody led to reduced platelet deposition and fibrin formation at the sites of injury in mouse vessels.

## Claims

1. A polypeptidic compound capable of blocking ICAM-4 binding to platelet integrin α_{IIb}β₃ for use in the treatment of a thrombotic disease, when administered to an individual diagnosed with such a condition.

2. A polypeptidic compound for use in the treatment of a thrombotic disease according to claim 1, which is selected from the group of:
- an anti-ICAM-4 antibody or a fragment thereof capable of blocking ICAM-4 binding to platelet integrin α_{IIb}β₃; and
- an ICAM-4 mimetic peptide capable of blocking ICAM-4 binding to platelet integrin α_{IIb}β₃; and
- an anti-CD61 antibody or a fragment thereof capable of blocking ICAM-4 binding to platelet integrin α_{IIb}β₃.

3. A polypeptidic compound according to claim 1 or 2 for use either for inhibiting erythrocyte-platelet adhesion, or for inhibiting platelet deposition and fibrin formation at the site of injury, or for reducing fibrinogen binding to integrin α_{IIb}β₃ on platelets.

4. The anti-CD61 antibody or fragment thereof for use according to claim 2, which is used in combination with the ICAM-4 mimetic peptide, or with the anti-ICAM-4 antibody or fragment thereof as defined in claim 2.

5. The ICAM-4 mimetic peptide for use according to claim 2, which is a synthetic peptide having the amino acid sequence Gly-Leu-Asp-Leu-Ala-Asn-Val-Thr-Leu-Thr-Tyr-Glu-Phe-Ala-Ala-Gly-Pro-Arg-Asp (GLDLANVTLTYEFAAGPRD) as disclosed in SEQ ID NO: 1.

6. A screening method to detect or monitor *in vitro* the effect of a substance, drug or pharmaceutical agent on the ICAM-4/α_{IIb}β₃ interaction in a biological sample, while simulating blood flow conditions existing *in vivo,* comprising the following steps:
a) mixing washed platelets and washed erythrocytes previously isolated from the biological sample,
b) pre-incubating the erythrocyte/platelet mixture with a platelet agonist for 2 minutes at 37°C,
c) carrying out the perfusion of the erythrocyte/platelet mixture on a platelet adhesive protein coated surface in a perfusion chamber under a venous flow shear rate for 5 minutes at 37°C,
d) acquiring images under a venous flow shear rate under differential interference contrast (DIC) microscopy,
e) quantifying the images to calculate the erythrocyte adherence.

7. The screening method according to claim 6, wherein the biological sample is selected from the group consisting of whole blood, plasma, or a derivative that would provide erythrocytes and platelets.

8. The screening method according to claim 6 or 7, wherein the platelet agonist of step b) is selected from the group consisting of collagen-related peptide, thrombin, ADP and arachidonic acid.

9. The screening method according to any one of claims 6 to 8, wherein the venous flow shear rate is within the range of 100 to 300 s⁻¹.

10. The screening method according to any one of claims 6 to 9, wherein the platelet adhesive protein is von Willebrand factor (VWF) or fibrinogen.

11. The screening method according to any one of claims 6 to 10, wherein said substance, drug or pharmaceutical agent is added to the biological sample to be assayed or is present in the biological sample as a result of the patient being under treatment with this substance, drug or pharmaceutical agent.

12. The screening method according to any one of claims 6 to 11 for use for detecting or monitoring a thrombotic disease.

13. The screening method according to any one of claims 6 to 12, wherein said substance, drug or pharmaceutical agent blocks the ICAM-4/α_{IIb}β₃ interaction.

14. The screening method according to any one of claims 6 to 13, wherein said substance, drug or pharmaceutical agent comprises a polypeptidic compound according to any one of claims 1 to 5.

15. The screening method according to any one of claims 6 to 14 for evaluating the risk of a patient to have a thrombotic condition or a bleeding phenotype, which comprises a step of measuring the expressed ICAM-4 in a biological sample previously obtained from a patient.

16. The screening method according to any one of claims 6 to 15, wherein an abnormally high ICAM-4 level is a risk indicator for a thrombotic disease, and an abnormally low ICAM-4 level is a risk indicator for a bleeding disease.

17. The screening method according to any one of claims 6 to 16, enabling the determination of the minimal amount of polypeptidic compound as defined in any one of claims 1 to 5 necessary to reduce or prevent thrombus formation, by blocking all or part of the ICAM-4/α_{IIb}β₃ interaction.

18. The screening method according to any one of claims 6 to 17, enabling the determination of the ICAM-4/α_{IIb}β₃ interaction by measuring thrombin generation.

19. A pharmaceutical composition comprising an anti-ICAM-4 antibody or a fragment thereof as defined in claim 2 or 3, or an ICAM-4 mimetic peptide as defined in any one of claims 2, 3 and 5, or an anti-CD61 antibody or a fragment thereof as defined in any one of claims 2 to 4, as active ingredient, in association with a pharmaceutically acceptable vehicle, and optionally a distinct therapeutically active molecule for use in the treatment of a thrombotic disease, when administered to an individual diagnosed with such a condition.

20. A kit for carrying out the screening method according to any one of claims 6 to 18, which comprises:
- an anti-ICAM-4 antibody or a fragment thereof as defined in claim 2 or 3, or an ICAM-4 mimetic peptide as defined in any one of claims 2, 3 and 5, or an anti-CD61 antibody or a fragment thereof as defined in any one of claims 2 to 4; and
- a platelet agonist.


SEQUENCE LISTING

<110>  SYNAPSE B.V.

<120>  Compounds and methods for inhibition of binding of ICAM-4 to
       platelet integrin ALPHAIIBBETA3

<130>  B10106 AD/DP/SBM

<160>  2

<170>  PatentIn version 3.5

<210>  1
<211>  19
<212>  PRT
<213>  Artificial Sequence

<220>
<223>  Synthetic peptide

<400>  1

Gly Leu Asp Leu Ala Asn Val Thr Leu Thr Tyr Glu Phe Ala Ala Gly
1               5                   10                  15

Pro Arg Asp

<210>  2
<211>  271
<212>  PRT
<213>  Homo sapiens

<220>
<221>  MISC_FEATURE
<222>  (1)..(271)
<223>  Intercellular adhesion molecule 4 (ICAM-4) (protein accession
       number UniProtKB/Swiss-Prot: Q14773.1)

<400>  2

Met Gly Ser Leu Phe Pro Leu Ser Leu Leu Phe Phe Leu Ala Ala Ala
1               5                   10                  15

Tyr Pro Gly Val Gly Ser Ala Leu Gly Arg Arg Thr Lys Arg Ala Gln
            20                  25                  30

Ser Pro Lys Gly Ser Pro Leu Ala Pro Ser Gly Thr Ser Val Pro Phe
        35                  40                  45

Trp Val Arg Met Ser Pro Glu Phe Val Ala Val Gln Pro Gly Lys Ser
    50                  55                  60

Val Gln Leu Asn Cys Ser Asn Ser Cys Pro Gln Pro Gln Asn Ser Ser
65                  70                  75                  80

Leu Arg Thr Pro Leu Arg Gln Gly Lys Thr Leu Arg Gly Pro Gly Trp
                85                  90                  95

Val Ser Tyr Gln Leu Leu Asp Val Arg Ala Trp Ser Ser Leu Ala His
            100                 105                 110

Cys Leu Val Thr Cys Ala Gly Lys Thr Arg Trp Ala Thr Ser Arg Ile
        115                 120                 125

Thr Ala Tyr Lys Pro Pro His Ser Val Ile Leu Glu Pro Pro Val Leu
    130                 135                 140

Lys Gly Arg Lys Tyr Thr Leu Arg Cys His Val Thr Gln Val Phe Pro
145                 150                 155                 160

Val Gly Tyr Leu Val Val Thr Leu Arg His Gly Ser Arg Val Ile Tyr
                165                 170                 175

Ser Glu Ser Leu Glu Arg Phe Thr Gly Leu Asp Leu Ala Asn Val Thr
            180                 185                 190

Leu Thr Tyr Glu Phe Ala Ala Gly Pro Arg Asp Phe Trp Gln Pro Val
        195                 200                 205

Ile Cys His Ala Arg Leu Asn Leu Asp Gly Leu Val Val Arg Asn Ser
    210                 215                 220

Ser Ala Pro Ile Thr Leu Met Leu Ala Trp Ser Pro Ala Pro Thr Ala
225                 230                 235                 240

Leu Ala Ser Gly Ser Ile Ala Ala Leu Val Gly Ile Leu Leu Thr Val
                245                 250                 255

Gly Ala Ala Tyr Leu Cys Lys Cys Leu Ala Met Lys Ser Gln Ala
            260                 265                 270